# EUROPÄISCHE PATENTSCHRIFT

(11) **EP 3 102 133 B1**
(45) Veröffentlichungstag und Bekanntmachung des Hinweises auf die Patenterteilung: **23.10.2024**
(21) Anmeldenummer: 15701978.7
(22) Anmeldetag: 02.02.2015
(51) Int. Cl.: A61B 18/14, A61B 18/00, A61B 17/00

(54) **MEDIZINISCHE VORRICHTUNG ZUR ABLATION VON GEWEBEZELLEN UND SYSTEM MIT EINER DERARTIGEN VORRICHTUNG**
MEDICAL DEVICE FOR ABLATING TISSUE CELLS AND SYSTEM COMPRISING A DEVICE OF THIS TYPE
DISPOSITIF MÉDICAL POUR L'ABLATION DE CELLULES DE TISSU ET SYSTÈME COMPORTANT UN TEL DISPOSITIF

(30) Priorität: 04.02.2014 DE 102014101348
(43) Veröffentlichungstag der Anmeldung: 14.12.2016
(73) Patentinhaber: ADMEDES GmbH, 75179 Pforzheim (DE); Acquandas GmbH, 24143 Kiel (DE)
(72) Erfinder: LIMA DE MIRANDA, Rodrigo, 24116 Kiel (DE); SCHEUERMANN, Torsten, 80803 München (DE)
(74) Vertreter: Meissner Bolte Partnerschaft mbB
(86) Internationale Anmeldenummer: PCT/EP2015/052026
(87) Internationale Veröffentlichungsnummer: WO 2015/117908

(56) Entgegenhaltungen:
- EP-A1- 2 732 784
- WO-A1-2010/056771
- WO-A1-2010/056771
- WO-A1-2014/205388
- WO-A1-2015/006480
- DE-A1- 102011 053 018
- DE-A1- 102011 053 018
- DE-A1- 102011 053 021
- US-A1- 2013 066 316
- US-A1- 2013 090 651
- US-A1- 2013 090 651
- US-A1- 2013 231 658
- US-A1- 2013 231 658

## Beschreibung

Die Erfindung betrifft eine medizinische Vorrichtung zur Ablation von Gewebezellen gemäß dem Oberbegriff des Patentanspruchs 1 sowie ein System mit einer derartigen Vorrichtung.

Aus der Praxis sind medizinische Vorrichtungen in Form von expandierbaren Ballonen bekannt, die in ein Körperhohlorgan eingeführt werden können. Die Ballone werden im Körperhohlorgan expandiert und gelangen so in Kontakt mit der Gefäßwand. Auf der Ballonoberfläche sind Elektroden angeordnet, die bei geeigneter Ansteuerung zu einer lokalen Temperaturerhöhung im angrenzenden Gewebe führen, wodurch Gewebe abgetragen wird. Der Gewebeabtrag bzw. die Ablation des Gewebes erfolgt bei Temperaturen über 50° C. Derartige Temperaturen führen zu einer Koagulation von Eiweißen und zu einer Nekrotisierung von Zellen.

Die bekannten ballonexpandierbaren Elektroden werden insbesondere zur Ablation von Gewebe in Blutgefäßen eingesetzt. Durch die Expansion des Ballons im Blutgefäß wird jedoch der Blutfluss in stromabwärtsgelegene Blutgefäße blockiert. Dies führt zu einer Nährstoffminderversorgung in stromabwärtsgelegenen Gewebearealen, was langfristig zu organischen Schäden führen kann. Ferner besteht bei den bekannten Vorrichtungen die Gefahr der Überhitzung, wodurch ebenfalls zusätzliche Schäden im umliegenden Gewebe verursacht werden können.

Aus US 2012/029500 A1 ist eine medizinische Vorrichtung mit einem Katheter bekannt, der an seinem distalen Ende eine expandierbare Gitterstruktur aufweist. Die Gitterstruktur dient als Träger für eine Elektrodenanordnung, wobei zwischen mehreren Elektroden Temperatursensoren angeordnet sind. Mit Hilfe der Temperatursensoren wird die beim Energieeintrag in das Gewebe erzeugte Temperatur erfasst und in Abhängigkeit davon die Ablationsstärke gesteuert. Die Elektroden und die Temperatursensoren sind auf der Gitterstruktur der bekannten Vorrichtung angeordnet. Das führt dazu, dass die Wandstärke der Gitterstruktur stellenweise vergrößert ist, was einerseits die Expansionsfähigkeit der Gitterstruktur beeinträchtigt und andererseits den Einsatz der Vorrichtung in kleinen Blutgefä-ßen erschwert.

Aus DE 10 2011 053 021 A1, der DE 10 2011 053 018 A1 und der US 2013/0066316 A1 sind Stentelektroden mit einer Gitterstruktur bekannt, auf deren Stegen Elektroden angeordnet sind. Die Elektroden sind derart mit den Stegen verbunden, dass sie die Flexibilität der Stege beeinflussen. Dadurch ist die bekannte Gitterstruktur schwer komprimierbar, zumal die Elektroden beim Komprimieren der Gitterstruktur nicht aus der Wandungsebene der Stege ausweichen können.

Des Weiteren zeigen die US 2013/0231658 A1 und die US 2013/0090651 A1 eine Stentelektrode mit einer Gitterstruktur. Nachteilig ist jedoch, dass die Temperatur im durch die Elektrode erhitzten Gewebe nicht überwacht werden kann.

In der WO 2015/006480 A1 ist eine Stentelektrode mit einer Gitterstruktur offenbart, deren Stege durch eine Beschichtung oder eine vergleichbare Verbindung mit einer oder mehrerer Elektrodeneinheiten verbunden sind. Dadurch resultiert eine instabile Verbindung der Elektrodeneinheiten mit der Gitterstruktur.

Die WO 2014/205388 A1 und die EP 2 732 784 A offenbaren ferner eine expandierbare medizinische Vorrichtungen mit Elektroden, deren Aufbau jedoch für den Einsatz in kleinen Gefäßen nicht bzw. nur eingeschränkt geeignet ist.

Aus der WO 2010/056771 A1 geht eine Elektrodenanordnung für minimalinvasive Behandlungen hervor. Nachteilig ist jedoch, dass die Elektrodenanordnung nicht bzw. nur eingeschränkt auf einer expandierbaren Gitterstruktur, die zum Einführen in ein Blutgefäß geeignet ist, angeordnet werden kann.

Die Aufgabe der Erfindung besteht darin, eine medizinische Vorrichtung zur Ablation von Gewebezellen anzugeben, die einerseits die Nährstoffzufuhr in stromabwärts gelegene Gewebeareale während der Ablation weitgehend aufrechterhält und andererseits für den Einsatz in kleinen Blutgefäßen ausreichend komprimierbar ist. Ferner besteht die Aufgabe der Erfindung darin, ein System mit einer derartigen Vorrichtung anzugeben.

Erfindungsgemäß wird diese Aufgabe im Hinblick auf die medizinische Vorrichtung durch den Gegenstand des Patentanspruchs 1 und im Hinblick auf das System durch den Gegenstand des Patentanspruchs 14 gelöst.

So beruht die Erfindung auf dem Gedanken, eine medizinische Vorrichtung zur Ablation von Gewebezellen mit mindestens einer Elektrode, mindestens einem Temperatursensor und einer radial expandierbaren, zumindest abschnittsweise rohrförmigen Gitterstruktur anzugeben, die Zellen begrenzende Stege aufweist. Erfindungsgemäß weist die Gitterstruktur wenigstens ein Kontaktelement auf, das mit den Stegen in einer Wandungsebene angeordnet und mit den Stegen verbunden ist, wobei die Elektrode und der Temperatursensor auf dem Kontaktelement angeordnet sind und wobei das Kontaktelement 13 freitragend innerhalb einer übergeordneten Zelle 12 angeordnet und einstückig, insbesondere monolithisch, mit den Stegen 11 ausgebildet ist

Bei der Erfindung ist also in unmittelbarer Nähe zur Elektrode, die vorzugsweise zur Ablation entsprechend angesteuert wird, ein Temperatursensor angeordnet. Auf diese Weise lässt sich die Temperatur im durch die Elektrode erhitzten Gewebe präzise überwachen und die Stromzufuhr zur Elektrode entsprechend steuern. Eine Überhitzung des Gewebes kann so effizient vermieden werden.

Außerdem umfasst die erfindungsgemäße Vorrichtung eine im Wesentlichen rohrförmige Gitterstruktur. Die Rohrform und die in der Gitterstruktur ausgebildeten Zellen ermöglichen die Durchströmung der medizinischen Vorrichtung, sodass durch den Einsatz der medizinischen Vorrichtung der Blutstrom in einem Blutgefäß kaum behindert wird. So kann bereits während des Einsatzes der medizinischen Vorrichtung die Nährstoffversorgung für stromabwärtsgelegene Gewebeareale wiederhergestellt oder aufrechterhalten werden.

Die Gitterstruktur ist vorzugsweise in Dünnschichttechnik hergestellt. Insbesondere kann die Gitterstruktur durch physikalische Gasphasenabscheidung, insbesondere durch ein PVD-Verfahren, erhalten sein. Ferner können zur Herstellung der Gitterstruktur kombinierte Sputter-Ätz-Verfahren eingesetzt werden. Die Bildung der Gitterstruktur als Dünnschicht hat den Vorteil, dass die Gitterstruktur bei kleinen Dimensionen relativ hohe Radialkräfte aufbringen kann. Die Dünnschichttechnik eignet sich zur Miniaturisierung der medizinischen Vorrichtung, sodass diese gut in kleinen Gefäßen, beispielsweise Hirngefäßen, einsetzbar ist. Die Gitterstrukturen, die durch Dünnschichttechnik hergestellt sind, zeichnen sich durch geringe Gitterbaufehler aus, was die mechanischen Eigenschaften der Gitterstruktur erheblich verbessert. Dies gilt insbesondere für die Bruchfestigkeit und die Flexibilität der Gitterstruktur. Das Kontaktelement, die Elektrode und der Temperatursensor können ebenfalls in Dünnschichttechnik hergestellt sein. Die Elektrode und der Temperatursensor können beispielsweise als zusätzliche Schicht auf die Dünnschicht der Gitterstruktur aufgebracht sein.

Bei der Erfindung ist das Kontaktelement in einer Wandungsebene mit den Stegen angeordnet. Das Kontaktelement trägt somit nicht zusätzlich zur Wandstärke der Gitterstruktur bei, sondern ist vielmehr in die Gitterstruktur integriert. Indem die Elektrode und der Temperatursensor auf dem Kontaktelement angeordnet bzw. in das Kontaktelement integriert sind, wird durch die Elektrode und den Temperatursensor die Expansion der Gitterstruktur kaum beeinflusst. Die mechanischen Eigenschaften der Gitterstruktur bleiben also weitgehend bestehen. Ferner wird durch die Integration des Kontaktelements in die Wandungsebene der Gitterstruktur erreicht, dass die medizinische Vorrichtung im Gebrauch ein relativ großes Innenlumen aufweist und daher gut durchströmbar ist. So kann während der Ablationsbehandlung ein ausreichend hoher Blutfluss durch ein Blutgefäß aufrechterhalten werden.

Bei der erfindungsgemäßen medizinischen Vorrichtung ist das Kontaktelement einstückig, insbesondere monolithisch, mit den Stegen ausgebildet. Das Kontaktelement kann in die Dünnschicht der Gitterstruktur integriert sein bzw. Teil der Dünnschicht sein, die sowohl die Gitterstruktur, als auch das Kontaktelement bildet. Die einstückige Herstellung von Kontaktelement und Gitterstruktur vereinfacht den Herstellungsprozess und verbessert die mechanischen Eigenschaften der gesamten medizinischen Vorrichtung.

Zumindest das Kontaktelement kann wenigstens abschnittsweise mit einer Isolationsschicht ummantelt sein, die im Bereich der Elektrode unterbrochen ist. Konkret kann die Isolationsschicht auf einer Außenfläche des Kontaktelements unterbrochen sein, sodass die Elektrode freiliegt. Die Isolationsschicht bewirkt eine elektrische Isolierung des Kontaktelements, sodass eine Ablation bzw. Koagulation von umliegendem Gewebe außerhalb der Elektroden vermieden wird. Die Isolationsschicht kann nur im Bereich des Kontaktelements angeordnet sein. Es ist auch möglich, dass sich die Isolationsschicht über zumindest einen Teil der Stege der Gitterstruktur erstreckt. Ferner ist denkbar, dass alle Stege und Kontaktelemente der Gitterstruktur durch eine Isolationsschicht ummantelt sind.

Das Kontaktelement kann im Allgemeinen innerhalb einer Zelle der Gitterstruktur angeordnet sein. Damit ist sichergestellt, dass das Kontaktelement die mechanischen Eigenschaften, insbesondere hinsichtlich der Expansionsfähgikeit, der Gitterstruktur nicht negativ beeinflusst. So kann die Gitterstruktur beispielsweise eine übliche Stentstruktur aufweisen, wobei das Kontaktelement zusätzlich vorgesehen ist. Durch die Anordnung des Kontaktelements innerhalb einer Zelle wird außerdem ein guter Kontakt mit anliegendem Gewebe erreicht, das durch die Radialkraft der Gitterstruktur in die Zellen eindringt.

In einer weiteren bevorzugten Ausführungsform der erfindungsgemäßen Vorrichtung weist die Gitterstruktur wenigstens eine elektrische Leiterbahn auf, die die Elektrode und/oder den Temperatursensor mit einem Strom- und/oder Datenkabel verbindet. Die elektrische Leiterbahn kann auf einer Innenseite der Gitterstruktur angeordnet sein, um den Abstand der Leiterbahn vom zu behandelnden Gewebe zu erhöhen. Die Anordnung der Leiterbahn auf dem Innenumfang der Gitterstruktur stellt selbst bei einer Beschädigung der Isolierung sicher, dass eine gezielte Gewebeerwärmung ausschließlich im Bereich der Elektroden am Kontaktelement erfolgt. So kann eine präzise Gewebeablation erreicht werden. Da die elektrische Verbindung zwischen der Elektrode und dem Strom- und/oder Datenkabel über in die Vorrichtung integrierte Leiterbahnen an der Gitterstruktur erfolgt, werden zusätzliche Leiter vermieden, die zu einer Beeinträchtigung des Blutflusses führen könnten. Dies verbessert die Rekanalisationsfunktion der Vorrichtung.

Ferner kann die medizinische Vorrichtung auf diese Weise leicht vom Strom- und/oder Datenkabel abgekoppelt werden und gegebenenfalls für eine weitere, langfristige Therapie, im Körper verbleiben. Beispielsweise kann die medizinische Vorrichtung als Stent ausgebildet sein, der nach der Gewebeablation zur Stützung des Blutgefäßes im Körper verbleibt. Somit erfüllt die Vorrichtung eine Doppelfunktion, einerseits hinsichtlich der Gewebeablation und andererseits hinsichtlich der Stütz- bzw. Rekanalisationsfunktion. Die Hauptaufgabe der stentartigen Struktur bzw. der Gitterstruktur besteht darin, einen ausreichenden Anpressdruck bereitzustellen, so dass ein guter Kontakt zwischen der Elektrode und dem Gewebe sichergestellt ist. Dies erhöht die Effizienz der Behandlung. Insbesondere wird auf diese Weise vermieden, dass Ablationsenergie, beispielsweise in Form von Wärme, dissipiert und somit die für die Ablation erforderliche Eindringtiefe nicht erreicht wird.

Im Allgemeinen ist darauf hinzuweisen, dass bei Verwendung einer Gitterstruktur als Träger für die Elektroden im Unterschied zu beispielsweise expandierbaren Ballonen die Einsatzmöglichkeiten erhöht sind. Eine Gitterstruktur weist üblicherweise einen größeren Durchmesserbereich zwischen dem komprimierten Durchmesser und dem expandierten Durchmesser auf. Daher ist eine Gitterstruktur für eine größere Anzahl unterschiedlich großer Gefäße einsetzbar. Der sogenannte "intended-use"-Bereich ist also bei der erfindungsgemäßen Vorrichtung gegenüber bekannten ballonkatheterbasierten Systemen erhöht.

Das Strom- und/oder Datenkabel kann innerhalb eines Transportdrahtes angeordnet sein. Mit anderen Worten kann ein Transportdraht für die Vorrichtung vorgesehen sein, der flexibel oder schlauchartig ausgebildet ist (Hypotube). Der Transportdraht dient einerseits dazu, die medizinische Vorrichtung an den Behandlungsort zu befördern. Gleichzeitig kann der Transportdraht als Führung für das Strom- und/oder Datenkabel genutzt werden. So ist das Strom- und/oder Datenkabel bei der Zuführung durch den Katheter durch den Transportdraht geschützt.

Die elektrische Leiterbahn der Gitterstruktur verläuft vorzugsweise entlang von Stegen der Gitterstruktur und ist in die Isolationsschicht eingebettet. Dabei kann die elektrische Leiterbahn vollständig von der Isolationsschicht ummantelt sein. Im Allgemeinen kann die medizinische Vorrichtung einen Schichtaufbau aufweisen, wobei die Gitterstruktur durch eine Tragschicht, die als Dünnschicht ausgebildet sein kann, gebildet ist. Die Tragschicht kann durch eine Isolationsschicht ummantelt sein. Auf der Isolationsschicht kann eine elektrische Leiterbahn als Leitungsschicht angeordnet sein, die wiederum durch eine weitere Isolationsschicht bedeckt ist. Die Leiterbahn bzw. Leitungsschicht kann also in die Isolationsschicht eingebettet sein, wobei die Leitungsschicht auf der Isolationsschicht aufliegt und durch die Isolationsschicht gleichzeitig bedeckt ist. Mit anderen Worten kann die Leiterbahn vollständig von der Isolationsschicht umgeben sein. Wenn die Tragschicht der Gitterstruktur ein nicht-leitendes Material aufweist, kann die Leiterbahn auch unmittelbar auf der Tragschicht angeordnet und durch die Isolationsschicht bedeckt sein.

In einer bevorzugten Ausgestaltung der Erfindung ist der Temperatursensor durch ein Thermoelement, insbesondere ein Thermocouple, gebildet. Derartige Thermoelemente zeichnen sich durch eine hohe Genauigkeit und einfache Herstellung aus. Insbesondere ist ein Thermoelement, insbesondere ein Thermocouple, einfach im Wege der Dünnschichttechnik herstellbar. Durch die Herstellung des Thermoelements in Dünnschichttechnik wird außerdem der Bauraum minimiert, sodass das Thermoelement gut in den Schichtaufbau der Vorrichtung integrierbar ist. Alternativ zu einem Thermocouple kann auch ein Thermistor, beispielsweise ein PT 100- oder Ni100-Thermistor, eingesetzt werden.

Das Kontaktelement kann zwei v-förmig angeordnete Trägerstege aufweisen, die mit Stegen der Gitterstruktur verbunden sind, sodass das Kontaktelement freitragend innerhalb einer Zelle der Gitterstruktur angeordnet ist. Eine derartige Konstruktion des Kontaktelements verbessert die mechanischen Eigenschaften der Gitterstruktur, da die Trägerstege mit den Stegen der Gitterstruktur, mit welchen sie verbunden sind, gemeinsam eine Unterzelle der Gitterstruktur bilden. Das Kontaktelement ist dabei freitragend innerhalb der übergeordneten Zelle angeordnet, sodass das Kontaktelement sich gut an die Gefäßwand anlegen kann. Gleichzeitig kann das Kontaktelement durch Berührung mit Gewebe ausgelenkt werden, sodass der lokale Druck des Kontaktelements auf das Gewebe durch das Biegemoment des Trägerstegs definiert bzw. eingestellt wird. Auf diese Weise wird ein ausreichend hoher Anpressdruck für die Elektrode bereitgestellt, der eine effiziente Gewebeablation gewährleistet.

Die Gitterstruktur weist vorzugsweise Verbinder auf, die jeweils vier Stege miteinander verbinden. Im Allgemeinen können die Stege jeweils Zellen begrenzen, die eine im Wesentlichen rautenförmige Grundform aufweisen. Die einzelnen rautenförmigen Zellen sind an ihren Ecken miteinander verbunden, sodass sich die Gitterstruktur bildet. Die Verbindungsstellen werden im Rahmen der Anmeldung als Verbinder bezeichnet. Die Verbinder sind vorzugsweise einstückig mit den Stegen ausgebildet.

In diesem Zusammenhang wird darauf hingewiesen, dass sich die rautenförmige Grundform der Zellen im expandierten Zustand der Gitterstruktur zeigt. Im Allgemeinen wird die Geometrie der medizinischen Vorrichtung in der vorliegenden Anmeldung anhand des expandierten Zustands beschrieben soweit kein Hinweis auf eine besondere Konstruktion im komprimierten Zustand gegeben wird.

Das Kontaktelement kann direkt an einem Verbinder angeordnet sein. Mit anderen Worten kann sich das Kontaktelement ausgehend vom Verbinder zwischen zwei Stegen erstrecken und mit dem Verbinder gekoppelt sein, zu welchem die beiden Stege konvergieren. Auf diese Weise ist das Kontaktelement relativ starr mit der Gitterstruktur verbunden bzw. ausgebildet, sodass durch die Radialkraft der Gitterstruktur ein guter Kontakt der Elektroden mit dem umliegenden Gewebe gewährleistet ist.

Das Kontaktelement weist vorzugsweise wenigstens zwei Elektroden auf, die ein bipolares Elektrodenpaar bilden. Eine bipolare Ansteuerung der Elektroden ist vorteilhaft, da auf diese Weise eine sehr gezielte und präzise Ablation von Gewebe erreicht wird.

Der Temperatursensor ist vorzugsweise zwischen den zwei Elektroden angeordnet. Somit ist sichergestellt, dass der Temperatursensor in einem Bereich angeordnet ist, in welchem die höchsten Temperaturen auftreten. Die elektrische Felddichte ist zwischen den bipolaren Elektroden maximal, sodass hier die Temperaturerhöhung groß ist und schnell erfolgt. Durch die Temperaturmessung in diesem Bereich wird sichergestellt, dass auch die Spitzenwerte der erreichten Ablationstemperaturen unmittelbar erfasst und bei Überschreiten eines bestimmtes Grenzwertes angepasst werden können. Dazu sind der Temperatursensor und die Elektrode bzw. die Elektroden vorzugsweise mit einer Steuerung verbindbar, die die elektrische Ablation in Abhängigkeit der Temperaturentwicklung im behandelten Gewebe einstellt.

Hinsichtlich der verwendeten Materialien kann vorgesehen sein, dass die Gitterstruktur ein Formgedächtnismaterial aufweist. Als Formgedächtnismaterial kann insbesondere eine Nickel-Titan-Legierung, vorzugsweise Nitinol, eingesetzt werden. Die Gitterstruktur ist vorzugsweise selbst expandierend. Dabei ermöglicht das Formgedächtnismaterial eine schnelle und effiziente Expansion, wenn die Gitterstruktur der Körpertemperatur des Patienten ausgesetzt ist. Vorzugsweise ist das Formgedächtnismaterial derart angepasst, dass die Gitterstruktur bei Körpertemperatur den expandierten Zustand selbsttätig einnimmt.

Die Elektrode und/oder der Temperatursensor und/oder die Leiterbahn können ein biokompatibles, elektrisch leitendes Material, insbesondere Gold, aufweisen. In anderen Worten können alle elektrisch leitenden Elemente der medizinischen Vorrichtung durch ein elektrisch leitendes Material gebildet sein, das vorzugsweise ebenfalls biokompatibel ist. Die Verwendung von Gold ist bevorzugt, da damit gute elektrische und körperverträgliche Eigenschaften erreicht werden.

Grundsätzlich kann der Temperatursensor als Thermocouple oder als Thermistor ausgelegt sein. Bei Verwendung eines Thermistors ist es bevorzugt, zwei Goldelektroden vorzusehen, insbesondere wenn der Thermistor durch Bonden mit der Gitterstruktur verbunden wird. Bei Herstellung des Thermistors durch Dünnfilmtechnologie ist es zweckmäßig, den Thermistor aus Platin (PT100-Thermistor) oder Nickel (Ni100-Thermistor) herzustellen. Um ein verlässliches Messergebnis bei Verwendung eines Thermocouple zu erzielen, ist es vorteilhaft, die gesamte Leiterbahn von einem Steuergerät bis zum Thermocouple aus dem jeweiligen Thermocouplematerial herzustellen. Beispielsweise kann eine Nickel-KupferLegierung (50/50) zum Thermocouple führen, und anschließend in einen (reinen) Kupferleiter übergehen, der wieder zurück an das Steuergerät führt. Die Paarung aus einem Nickel-Kupfer-Element und einem Kupferelement bildet das Thermocouple.

Ein nebengeordneter Aspekt der Erfindung beruht auf dem Gedanken, ein System mit einer zuvor beschriebenen Vorrichtung und einer Zuführeinrichtung, insbesondere einem Katheter, anzugeben. Das System weist ferner einen Transportdraht auf, der längsverschieblich innerhalb des Katheters geführt ist. Der Transportdraht umfasst außerdem einen Durchgangskanal zur Aufnahme eines Strom- und/oder Datenkabels, das die Elektrode und/oder den Temperatursensor elektrisch mit einer Steuerung verbindet.

Die Erfindung wird im Folgenden anhand von Ausführungsbeispielen unter Bezugnahme auf die beigefügten, schematischen Zeichen näher erläutert. Darin zeigen:
- Fig. 1:: eine schematische Ansicht eines Systems mit der erfindungsgemäßen medizinischen Vorrichtung nach einem bevorzugten Ausführungsbeispiel;
- Fig. 2:: eine Detailansicht des Systems gemäß Fig. 1 bei der Expansion der Vorrichtung in einem Blutgefäß;
- Fig. 3:: eine perspektivische Ansicht der erfindungsgemäßen medizinischen Vorrichtung nach einem weiteren Ausführungsbeispiel;
- Fig. 4:: eine Detailansicht des Kontaktelements der Vorrichtung gemäß Fig. 3;
- Fig. 5:: eine Querschnittsansicht durch das Kontaktelement entlang der Linie A-A in Fig. 4;
- Fig. 6:: eine Schnittansicht durch ein Kontaktelement einer erfindungsgemäßen medizinischen Vorrichtung im Bereich der Elektroden und des Temperatursensors;
- Fig. 7:: eine Schnittansicht durch einen Steg der Gitterstruktur der erfindungsgemäßen medizinischen Vorrichtung, wobei in eine Isolationsschicht der Gitterstruktur Leiterbahnen eingebettet sind; und
- Fig. 8:: eine Draufsicht auf ein Kontaktelement der erfindungsgemäßen medizinischen Vorrichtung, zur Darstellung des Verlaufs der Leiterbahnen;

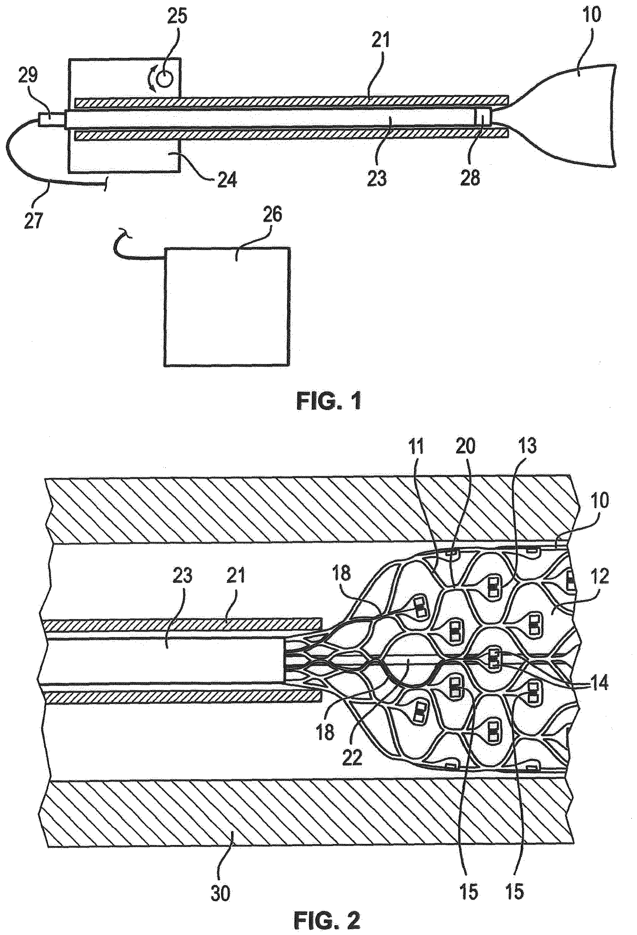

Fig. 1 zeigt ein System mit einer medizinischen Vorrichtung, die zur Ablation von Gewebezellen geeignet ist. Die medizinische Vorrichtung weist eine expandierbare Gitterstruktur 10 auf, die im expandierten Zustand im Wesentlichen eine rohrförmige Außenkontur aufweist. Die Gitterstruktur ist durch Stege 11 gebildet, die Zellen 12 begrenzen. Jeweils vier Stege 11 bilden dabei eine Zelle 12. Das System weist ferner einen Katheter 21 auf, der zur Zufuhr der Gitterstruktur 10 in ein Hohlorgan 30 dient. Der Katheter 21 ist mit einem Handgriff 24 verbunden. Der Handgriff 24 umfasst ein drehbares Rad 25, das mit einem Transportdraht 23 in Verbindung steht. Der Transportdraht 23 ist längsverschieblich in dem Katheter 21 angeordnet und kann über das Rad 25 durch den Katheter 21 geschoben oder gezogen werden.

Der Transportdraht 23 weist einen distalen Konnektor 28 auf, sodass der Transportdraht 23 elektrisch mit der Gitterstruktur 10 verbindbar ist. Ferner ist ein proximaler Konnektor 29 am Transportdraht 23 vorgesehen, der elektrisch mit einem Strom- und/oder Datenkabel 27 verbindbar ist. Das Strom- und/oder Datenkabel 27 führt zu einer Steuerung 26. Mit Hilfe des Strom- und/oder Datenkabels 27 sind also der Transportdraht 23 und somit auch die Gitterstrukturen 10 mit der Steuerung 26 verbindbar.

Wie in Fig. 1 schematisch dargestellt ist, ist die Gitterstruktur 10 vorzugsweise selbstexpandierbar, sodass sich die Gitterstruktur 10 radial ausweitet, sobald die Gitterstruktur 10 den Katheter 21 verlässt. Dabei kann sich die Gitterstruktur 10 an die Gefäßwand eines Hohlorgans 30 anlegen und so einerseits die Gefäßwand des Hohlorgans 30 stützen und andererseits durch die Zellen 12 gleichzeitig einen Durchfluss von beispielsweise Blut oder Gasen durch das Hohlorgan 30 ermöglichen.

Fig. 2 zeigt einen Detailausschnitt des Systems gemäß Fig. 1 bei Anordnung der Gitterstruktur 10 in dem Hohlorgan 30. Es ist gut erkennbar, dass die Gitterstruktur 10 durch den Transportdraht 23 an den Behandlungsort geführt wird. Insbesondere kann der Transportdraht 23 mit der Gitterstruktur 10 zumindest temporär verbindbar sein. Um den Transportdraht 23 und den Katheter 21 an den Behandlungsort zu führen, kann vorteilhaft ein Führungsdraht 22 eingesetzt werden. Der Führungsdraht 22 verläuft durch den Transportdraht 23, der als Hohlrohr ausgebildet ist. Innerhalb des Transportdrahts 23 kann auch das Strom- und/oder Datenkabel 27 angeordnet sein, das durch Konnektoren 28, 29 einerseits mit der Gitterstruktur 10 und andererseits mit der Steuerung 26 verbindbar ist.

Die Gitterstruktur 10 weist Stege 11 und Zellen 12 auf. Ferner umfasst die Gitterstruktur 10 Kontaktelemente 13, die innerhalb der Zellen 12 angeordnet sind. Bei dem Ausführungsbeispiel gemäß Fig. 2 sind die Kontaktelemente 13 einstückig mit den Stegen 11 ausgebildet. Insbesondere sind die Kontaktelemente 13 mit Verbindern 20 einstückig gekoppelt. Die Verbinder 20 verbinden jeweils vier Stege 11 bzw. bilden Kreuzungspunkte zwischen den vier Stegen 11.

Die Kontaktelemente 13 tragen jeweils zwei Elektroden 14. Dabei sind die Elektroden 14 auf einer Außenumfangsfläche der Gitterstruktur 10, insbesondere der Kontaktelemente 13, angeordnet. Mit anderen Worten umfasst jedes Kontaktelement 13 ein Elektrodenpaar aus jeweils zwei Elektroden 14. Die Elektroden 14 eines Elektrodenpaars bilden vorzugsweise eine bipolare Elektrodenanordnung. Zwischen den zwei Elektroden 14 ist an dem Kontaktelement 13 ein Temperatursensor 15 angeordnet. Der Temperatursensor 15 ist vorzugsweise ebenfalls auf der Außenfläche des Kontaktelements 13 angebracht.

Die Elektroden 14 und die Temperatursensoren 15 sind über Leiterbahnen 18 mit dem Strom- und/oder Datenkabel 27 verbunden. Die Leiterbahnen 18 erstrecken sich vorzugsweise entlang der Stege 11 der Gitterstruktur 10. Dabei verlaufen die Leiterbahnen 18 vorzugsweise auf einer Innenseite der Gitterstruktur 10. Insbesondere verlaufen die Leiterbahnen 18 auf einer Innenseite der Stege 11. Im Bereich der Kontaktelemente 13 können die Leiterbahnen 18 auch auf der Außenfläche verlaufen. Konkret ist vorgesehen, die Leiterbahnen 18 ausgehend von den Elektroden 14 bzw. den Temperatursensoren 15 im Bereich der Kontaktelemente 13 von der Außenfläche zur Innenfläche zu führen und anschließend entlang der Stege 11 auf der Innenfläche zum Strom- und/oder Datenkabel 27 bzw. zum Transportdraht 23 zu leiten.

Im Allgemeinen weist die Gitterstruktur 10 eine stentartige Struktur auf. Die Gitterstruktur 10 kann daher eine Doppelfunktion übernehmen. Einerseits kann die Gitterstruktur 10 durch die Elektroden 14 zur Ablation von Gewebe eingesetzt werden. Andererseits bewirkt die stentartige Struktur der Gitterstruktur 10 eine Radialkraft, sodass durch die Gitterstruktur 10 ein Hohlorgan 30 gestützt und gegebenenfalls aufgeweitet werden kann.

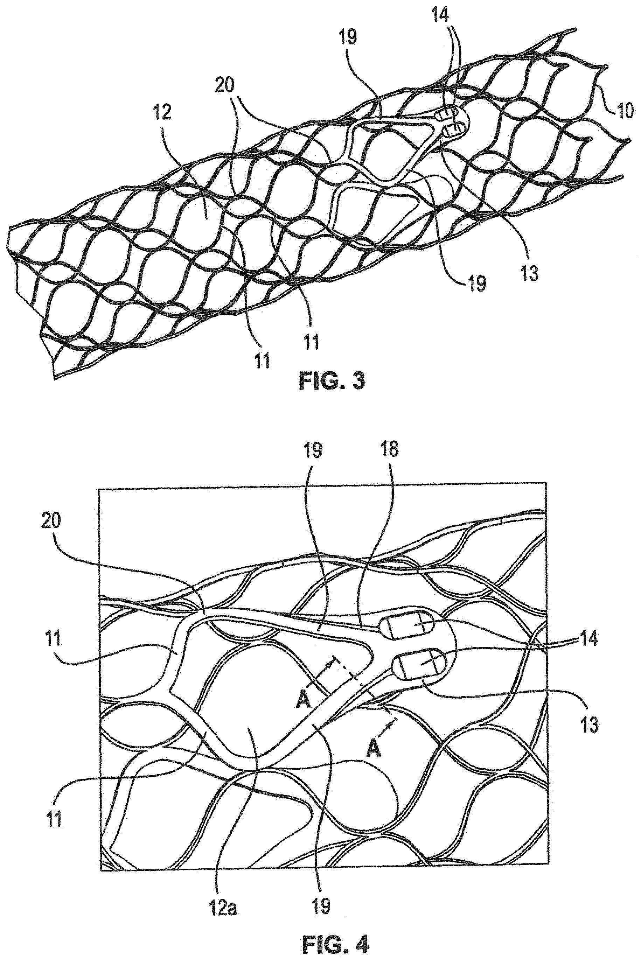

Fig. 3 zeigt eine alternative Ausführungsform der medizinischen Vorrichtung, wobei die Gitterstruktur 10 stentartig ausgebildet ist. Die Gitterstruktur 10 weist insbesondere Stege 11 auf, die Zellen 12 begrenzen. Das Kontaktelement 13 weist bei dem Ausführungsbeispiel gemäß Fig. 3 zwei Trägerstege 19 auf, die mit den Stegen 11 der Gitterstruktur 10 verbunden sind. Die Trägerstege 19 bilden mit den Stegen 11 der Gitterstruktur 10 insbesondere eine Unterzelle 12a. Die Trägerstege 19 verbinden also das Kontaktelement 13 mit den Stegen 11 der Gitterstruktur 10, wobei das Kontaktelement 13 im Wesentlichen freitragend innerhalb einer übergeordneten Zelle 12 angeordnet ist. Die Konstruktion des Kontaktelementes 13 gemäß Fig. 3 ist im Wesentlichen freischwingend, sodass das Kontaktelement 13 aus der Wandungsebene der Gitterstruktur 10 auslenkbar ist.

In Fig. 4 ist eine Detailansicht des Kontaktelements 13 gemäß Fig. 3 gezeigt. Gut erkennbar sind die beiden Elektroden 14, die auf der Außenfläche des Kontaktelements 13 angeordnet sind. Ferner ist erkennbar, dass entlang der Trägerstege 19 Leiterbahnen 18 verlaufen. Die Leiterbahnen 18 verlaufen entlang der Trägerstege 19 von der Elektrode 14 bzw. dem Temperatursensor 15 bis zu einem proximalen Ende der Gitterstruktur 10 und sind, ggf. über weitere elektrische Leiter, mit einem Steuergerät verbunden. Die Lage der Leiterbahnen kann entlang der Trägerstege 19 von der Außenseite der Gitterstruktur 10 zur Innenseite der Gitterstruktur 10 verlegt sein, sodass die Leiterbahnen 18 im Bereich der Stege 11 auf der Innenfläche der Gitterstruktur 10 angeordnet sind.

Das Kontaktelement 13 und die Stege 11 der Gitterstruktur 10 sowie die Leiterbahnen 18 sind durch eine Isolationsschicht 17 ummantelt. Die Isolationsschicht 17 kann mehrere Mikrometer dick sein und beispielsweise aus Parylene bestehen. Die Isolationsschicht 17 ist im Bereich der Elektroden 14 unterbrochen, sodass die Elektroden 14 direkten Kontakt zu umliegenden Gewebe erhalten.

Im Allgemeinen ist die Gitterstruktur 10 aus Dünnschichten aufgebaut. Insbesondere umfasst die Gitterstruktur 10 eine Tragschicht 16, die hauptsächlich die mechanische Stabilität der Gitterstruktur 10 bewirkt. Die Tragschicht 16 ist vorzugsweise aus einem Formgedächtnismaterial gebildet, beispielsweise einer Nickel-Titan-Legierung. Die Tragschicht ist von einer Isolationsschicht 17 ummantelt, die wesentlich dünner als die Tragschicht 16 ist. Die Isolationsschicht 17 bewirkt eine elektrische Isolation der Tragschicht 16. Vorzugsweise wird als Isolationsschicht 17 eine Oxidschicht eingesetzt.

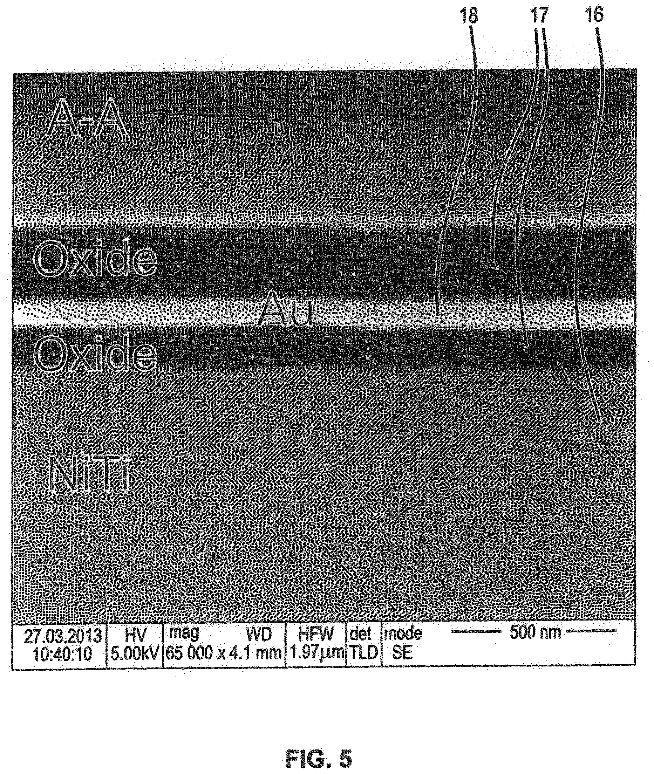

Fig. 5 zeigt beispielhaft einen Querschnitt durch einen Trägersteg 19 im Bereich der Linie A-A in Fig. 4. Darin ist erkennbar, dass die Tragschicht 16 durch eine Isolationsschicht 17 bedeckt ist. In die Isolationsschicht 17 ist eine Leiterbahn 18 eingebettet. Die Leiterbahn 18 umfasst ein elektrisch leitendes Material, das vorzugsweise auch biokompatibel ist. Ein geeignetes Material ist beispielsweise Gold. Die Leiterbahn 18 ist vorzugsweise vollständig durch die Isolationsschicht 17 ummantelt, sodass die Leiterbahn 18 von der Tragschicht 16 elektrisch isoliert ist.

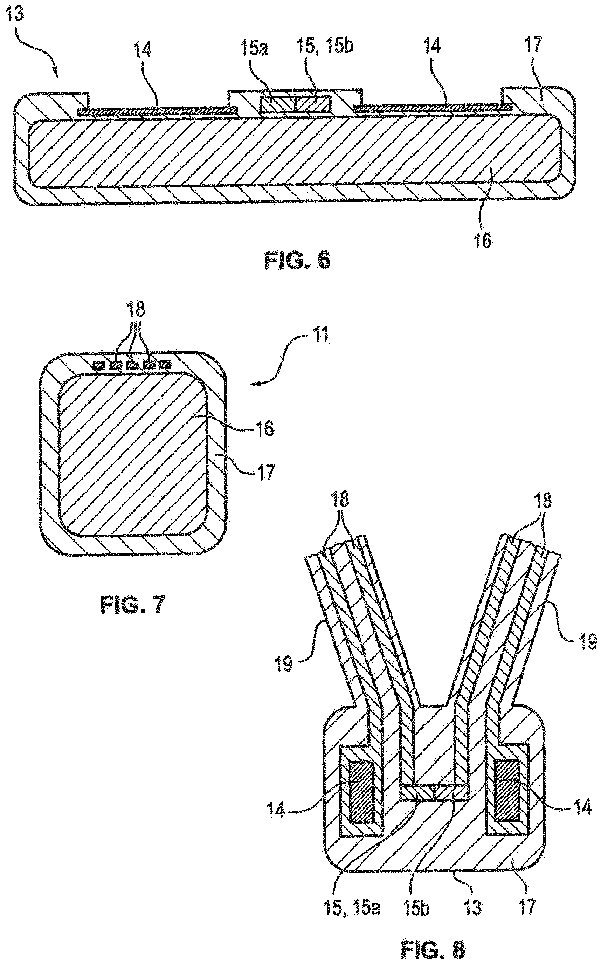

Fig. 6 zeigt einen Querschnitt durch das Kontaktelement 13 im Bereich der Elektroden 14. Es ist gut erkennbar, dass das Kontaktelement 13 ebenfalls die Tragschicht 16 der Gitterstruktur 10 aufweist. Die Tragschicht 16 ist durch eine Isolationsschicht 17 ummantelt, die im Bereich der Elektroden 14 unterbrochen ist. In die Isolationsschicht 17 ist ferner der Temperatursensor 15 eingebettet. Der Temperatursensor 15 ist dabei zwischen den Elektroden 14 angeordnet. Die Elektroden 14 bilden ein bipolares Elektrodenpaar.

Der Temperatursensor 15 kann beispielsweise als Thermoelement bzw. Thermocouple ausgebildet sein. Dazu weist der Temperatursensor vorzugsweise zwei unterschiedliche Materialkomponenten auf. Insbesondere kann ein Kupferelement 15a und ein Konstantanelement 15b vorgesehen sein. Derartige Thermoelemente werden als Typ-T-Thermoelemente bezeichnet. Diese sind für Messungen im Bereich von -200°C bis 350°C geeignet. Die Sensibilität derartiger Thermoelemente beträgt etwa 43 µV/°C bei einer Toleranz von +/- 0,5°C im Bereich zwischen - 40°C und 125°C. Das Kupferelement 15a ist dabei aus Kupfer gebildet, wogegen das Kontantanelement 15b aus Konstantan besteht, was eine Kupfer-NickelLegierung ist. Konstantan umfasst vorzugsweise 55% Kupfer und 45% Nickel. Die die einzelnen Materialkomponenten des Thermocouple mit dem Steuergerät verbindenden Leiterbahnen 18 bestehen jeweils aus demselben Material wie die jeweils zugehörige Materialkomponente, um ein elektrisches Spannungsgefälle zu erhalten und dieses korrekt auswerten zu können.

Fig. 7 zeigt einen Schnitt durch einen Steg 11 der Gitterstruktur 10. Der Steg 11 umfasst die Tragschicht 16 und eine Isolationsschicht 17, die die Tragschicht vollständig ummantelt. In die Tragschicht 17 sind mehrere Leiterbahnen 18 eingebettet. Die Leiterbahnen 18 können zu einzelnen Elektroden 14 und/oder Temperatursensoren 15 führen.

In Fig. 8 ist eine Draufsicht auf ein Kontaktelement 13 gezeigt, dass Trägerstege 19 zur Verbindung mit den Stegen 11 der Gitterstruktur 10 aufweist. Das Kontaktelement 13 und die Trägerstegen sind 19 einstückig mit den Stegen 11 der Gitterstruktur 10 ausgebildet.

In Fig. 8 ist gut erkennbar, dass von jeder Elektrode 14 jeweils eine Leiterbahn 18 abgeht und entlang der Trägerstege 19 verläuft. Außerdem sind zwei Leiterbahnen 18 vorgesehen, die mit dem Temperatursensor 15 verbunden sind. Dabei ist eine Leiterbahn 18 mit dem Kupferelement 15a und eine weitere Leiterbahn 18 mit dem Konstantanelement 15b gekoppelt. Die Leiterbahnen 18 bewirken eine elektrische Verbindung der Elektroden 14 und des Temperatursensors 15 mit einer Steuerung 26, sodass die Stromzufuhr zu den Elektroden 14 anhand der durch den Temperatursensor 15 gemessenen Temperatur eingestellt werden kann. In Fig. 8 ist außerdem gut erkennbar, dass die Isolationsschicht 17 im Bereich der Elektroden 14 unterbrochen ist. Zumindest ein Teil der Elektroden, insbesondere eine mittlere Fläche der Elektroden 14 ist also zur Umgebung hin frei und kann in Kontakt mit dem Hohlorgan 30 treten.

Im Hinblick auf den Temperatursensor 15 ist vorgesehen, diesen als Thermoelement bzw. Thermocouple auszubilden. Besonders bevorzugt ist ein Thermoelement des Typ T, das aus einem Kupferelement 15a und einem Konstantanelement 15b besteht. Andere Thermoelemente sind ebenfalls nutzbar. Eine Übersicht über die nutzbaren Thermoelemente mit unterschiedlichen Materialkombinationen zeigt die nachfolgende Tabelle:

| **Type** | **Material** | **Temperatur-** | **Temperatur-** |
|---|---|---|---|
| | | **Bereich (°C)** | **Bereich (°C)** |
| K | Ni-CrNi | 0 bis +1100 | -180 bis +1300 |
| J | Fe-CuNi | 0 bis +750 | -180 bis +800 |
| N | NiCrSi-NiSi | 0 bis +1100 | -270 bis +1300 |
| R | Pt13Rh-Pt | 0 bis +1600 | -50 bis +1700 |
| S | Pt10Rh-Pt | 0 bis +1600 | -50 bis +1750 |
| B | Pt30Rh-Pt6Rh | +200 bis +1700 | 0 bis +1820 |
| T | Cu-CuNi | -185 bis +300 | -250 bis +400 |
| E | NiCr-CuNi | 0 bis +800 | -40 bis +900 |
| | ChromeI-AuFe | -272 bis +300 | |

Bei Verwendung eines Thermistors als Temperatursensor 15 ist es möglich, einen Pol des Thermistors auf Masse zu legen. Ebenso kann bei Einsatz von bipolaren Elektrodenpaaren eine der Elektroden 14 eines Elektrodenpaars auf Masse gelegt werden. Somit können Leiterbahnen 18 eingespart werden, was die Herstellung der medizinischen Vorrichtung erleichtert.

Die zuvor beschriebene medizinische Vorrichtung ist insbesondere zur Ablation von Gewebe in Blutgefäßen geeignet, insbesondere im zerebralen Bereich. Es ist auch möglich, die medizinische Vorrichtung im Bereich der Trachea, der Bronchen, des Ösophagus oder in anderen Bereichen des gastrointestinalen Trakts einzusetzen. Insbesondere eignet sich die Erfindung dazu, Nervenbahnen im intravaskulären, pulmonalen oder gastrointestinalen Bereich thermisch zu veröden. Ferner kann die Erfindung zur Ablation von Nervenbahnen im Bereich der Nierenarterien eingesetzt werden, beispielsweise um Bluthochdruck zu behandeln.

Die erfindungsgemäße medizinische Vorrichtung hat mehrere Vorteile. Einerseits wird durch die Gitterstruktur 10 eine Unterbrechung des Blutflusses oder des Gasflusses in Körperhohlorganen vermieden. Durch die Verwendung von Dünnschichttechniken kann die Gitterstruktur 10 sehr klein gestaltet werden, sodass sich die medizinische Vorrichtung durch kleine Katheter 21 an den Behandlungsort führen lässt. Insbesondere kann die Gitterstruktur 10 derart dimensioniert sein, dass sie durch Katheter 21 oder andere Zuführeinrichtungen an den Behandlungsort führbar ist, die eine Größe von weniger als 5 French (intravaskuläre Anwendung) oder einen Innendurchmesser von weniger als 2,5 mm (Zuführung über ein Bronchoskop) aufweisen. Mit Hilfe des Temperatursensors wird eine Überhitzung des umliegenden Gewebes vermieden. Außerdem wird durch die Gitterstruktur 10 und die Anordnung der Kontaktelemente 13 sichergestellt, dass die Elektroden 14 vollständig Kontakt zum umliegenden Gewebe erhalten. Das Ablationsverfahren ist mit der erfindungsgemäßen medizinischen Vorrichtung in einem kurzen Zeitraum bei minimaler Energiezufuhr durchführbar.

### Bezugszeichenliste

- 10: Gitterstruktur
- 11: Steg
- 12: Zelle
- 12a: Unterzelle
- 13: Kontaktelement
- 14: Elektrode
- 15: Temperatursensor
- 15a: Kupferelement
- 15b: Konstantanelement
- 15c: Widerstandselement
- 16: Tragschicht
- 17: Isolationsschicht
- 18: Leiterbahn
- 19: Trägersteg
- 20: Verbinder
- 21: Katheter
- 22: Führungsdraht
- 23: Transportdraht
- 24: Handgriff
- 25: Rad
- 26: Steuerung
- 27: Strom- und/oder Datenkabel
- 28: distaler Konnektor
- 29: proximaler Konnektor
- 30: Hohlorgan

## Patentansprüche

1. Medizinische Vorrichtung zur Ablation von Gewebezellen mit mindestens einer Elektrode (14), mindestens einem Temperatursensor (15) und einer radial expandierbaren, zumindest abschnittsweise rohrförmigen Gitterstruktur (10), die Zellen (12) begrenzende Stege (11) aufweist,
wobei die Gitterstruktur (10) wenigstens ein Kontaktelement (13) aufweist, das in einer Wandungsebene mit den Stegen (11) angeordnet ist und mit den Stegen (11) verbunden ist, wobei die Elektrode (14) und der Temperatursensor (15) auf dem Kontaktelement (13) angeordnet sind,
**dadurch gekennzeichnet, dass**
das Kontaktelement (13) freitragend innerhalb einer übergeordneten Zelle (12) angeordnet und einstückig, insbesondere monolithisch, mit den Stegen (11) ausgebildet ist.

2. Vorrichtung nach Anspruch 1, wobei zumindest das Kontaktelement (13) wenigstens abschnittsweise mit einer Isolationsschicht (17) ummantelt ist, die im Bereich der Elektrode (14) unterbrochen ist.

3. Vorrichtung nach einem der vorhergehenden Ansprüche, wobei das Kontaktelement (13) innerhalb einer Zelle (12) der Gitterstruktur (10) angeordnet ist.

4. Vorrichtung nach einem der vorhergehenden Ansprüche, wobei die Gitterstruktur (10) wenigstens eine elektrische Leiterbahn (18) aufweist, die die Elektrode (14) und/oder den Temperatursensor (15) mit einem Strom- und/oder Datenkabel (27) verbindet.

5. Vorrichtung nach Anspruch 4, wobei das Strom- und/oder Datenkabel (27) innerhalb eines Transportdrahts (23) angeordnet ist.

6. Vorrichtung nach Anspruch 4 oder 5, wobei die elektrische Leiterbahn (18) entlang von Stegen (11) der Gitterstruktur (10) verläuft und in die Isolationsschicht (17) eingebettet ist.

7. Vorrichtung nach einem der vorhergehenden Ansprüche, wobei der Temperatursensor (15) durch ein Thermoelement, insbesondere ein Thermocouple, gebildet ist.

8. Vorrichtung nach einem der vorhergehenden Ansprüche, wobei das Kontaktelement (13) zwei v-förmig angeordnete Trägerstege (19) aufweist, die mit Stegen (11) der Gitterstruktur (10) verbunden sind derart, dass das Kontaktelement (13) freitragend innerhalb einer Zelle (12) der Gitterstruktur (10) angeordnet ist.

9. Vorrichtung nach einem der Ansprüche 1 bis 7, wobei die Gitterstruktur (10) Verbinder (20) aufweist, die jeweils vier Stege (11) miteinander verbinden, wobei das Kontaktelement (13) direkt an einem Verbinder (20) angeordnet ist.

10. Vorrichtung nach einem der vorhergehenden Ansprüche, wobei das Kontaktelement (13) wenigstens zwei Elektroden (14) aufweist, die ein bipolares Elektrodenpaar bilden.

11. Vorrichtung nach Anspruch 9, wobei der Temperatursensor (15) zwischen den zwei Elektroden (14) angeordnet ist.

12. Vorrichtung nach einem der vorhergehenden Ansprüche, wobei die Gitterstruktur (10) ein Formgedächtnismaterial, insbesondere eine Nickel-Titan-Legierung, vorzugsweise Nitinol, aufweist.

13. Vorrichtung nach einem der vorhergehenden Ansprüche, wobei die Elektrode (14) und/oder der Temperatursensor (15) und/oder die Leiterbahn (18) ein biokompatibles, elektrisch leitendes Material, insbesondere Gold, aufweist.

14. System mit einer Vorrichtung nach einem der vorhergehenden Ansprüche und einer Zuführeinrichtung, insbesondere einem Katheter (21), und einem Transportdraht (23), wobei der Transportdraht (23) längsverschieblich innerhalb des Katheters (21) geführt ist und einen Durchgangskanal zur Aufnahme eines Strom- und/oder Datenkabels (27) aufweist, das die Elektrode (14) und/oder den Temperatursensor (15) elektrisch mit einer Steuerung (26) verbindet.

## Claims

1. Medical device for ablating tissue cells, having at least one electrode (14), at least one temperature sensor (15) and a radially expandable, at least partially tubular mesh structure (10) that has struts (11) delimiting the cells (12),
wherein the mesh structure (10) has at least one contact element (13) which is arranged in a wall plane with the struts (11) and is connected to the struts (11), wherein the electrode (14) and the temperature sensor (15) are arranged on the contact element (13),
**characterized in that**
the contact element (13) is arranged in self-supporting manner inside a higher level cell (12) and is designed integrally, in particular monolithically, with the struts (11).

2. Device according to Claim 1,
wherein
at least the contact element (13) is at least partially surrounded with an insulating layer (17), which is interrupted in the region of the electrode (14).

3. Device according to one of the preceding claims,
wherein
the contact element (13) is arranged inside a cell (12) of the mesh structure (10).

4. Device according to any one of the preceding claims,
wherein
the mesh structure (10) has at least one electrical conductor track (18) which connects the electrode (14) and/or the temperature sensor (15) to a power and/or data cable (27).

5. Device according to Claim 4,
wherein
the power and/or data cable (27) is arranged inside a transport wire (23).

6. Device according to Claim 4 or 5,
wherein
the electrical conductor track (18) runs along struts (11) of the mesh structure (10) and is embedded in the insulating layer (17).

7. Device according to any one of the preceding claims,
wherein
the temperature sensor (15) is formed by a thermoelement, in particular a thermocouple.

8. Device according to any one of the preceding claims,
wherein
the contact element (13) has two carrier struts (19) arranged in v-formation, which are connected to struts (11) of the mesh structure (10), such that the contact element (13) is arranged in self-supporting manner inside a cell (12) of the mesh structure (10).

9. Device according to any one of Claims 1 to 7,
wherein
the mesh structure (10) contains connectors (20) which connect four struts (11) to each other in each case, the contact element (13) being arranged directly on a connector (20).

10. Device according to any one of the preceding claims,
wherein
the contact element (13) includes at least two electrodes (14) which form a bipolar electrode pair.

11. Device according to Claim 9,
wherein
the temperature sensor (15) is arranged between the two electrodes (14).

12. Device according to any one of the preceding claims,
wherein
the mesh structure (10) comprises a shape memory material, in particular a nickel-titanium alloy, preferably nitinol.

13. Device according to any one of the preceding claims,
wherein
the electrode (14) and/or the temperature sensor (15) and/or the conductor track (18) comprises a biocompatible, electrically conductive material, particularly gold.

14. System with a device according to any one of the preceding claims and a delivery device, in particular a catheter (21), and a transport wire (23), wherein the transport wire (23) is guided inside the catheter (21) in longitudinally displaceable manner, and a passthrough channel to accommodate a power and/or data cable (27) which connects the electrode (14) and/or the temperature sensor (15) to a controller (26).

## Revendications

1. Dispositif médical, destiné à l'ablation de cellules tissulaires, pourvu d'au moins une électrode (14), d'au moins un capteur de température (15) et d'une structure en treillis (10) expansible en direction radiale, de forme tubulaire au moins par endroits, qui comporte des barrettes (11) qui délimitent des cellules (12),
la structure en treillis (10) comportant au moins un élément de contact (13) qui est placé dans un plan de paroi avec les barrettes (11) et qui est connecté avec les barrettes (11), l'électrode (14) et le capteur de température (15) étant placés sur l'élément de contact (13), **caractérisé en ce que** l'élément de contact (13) est placé en étant autoportant à l'intérieur d'une cellule (12) d'ordre supérieur et est conçu en monobloc, notamment de manière monolithique avec les barrettes (11).

2. Dispositif selon la revendication 1, au moins l'élément de contact (13) étant enveloppé au moins par endroits d'une couche isolante (17) qui est interrompue dans la zone de l'électrode (14).

3. Dispositif selon l'une quelconque des revendications précédentes, l'élément de contact (13) étant placé à l'intérieur d'une cellule (12) de la structure en treillis (10).

4. Dispositif selon l'une quelconque des revendications précédentes, la structure en treillis (10) comportant au moins une piste conductrice (18) électrique, qui connecte l'électrode (14) et / ou le capteur de température (15) avec un câble électrique et / ou un câble de données (27).

5. Dispositif selon la revendication 4, le câble électrique et / ou le câble de données (27) étant placé à l'intérieur d'un fil de transport (23).

6. Dispositif selon la revendication 4 ou 5, la piste conductrice (18) électrique s'étendant le long des barrettes (11) de la structure en treillis (10) et étant incorporée dans la couche isolante (17).

7. Dispositif selon l'une quelconque des revendications précédentes, le capteur de température (15) étant constitué par un élément thermique, notamment par un thermocouple.

8. Dispositif selon l'une quelconque des revendications précédentes, l'élément de contact (13) comportant deux barrettes de support (19) placées en forme de V, qui sont connectées avec des barrettes (11) de la structure en treillis (10) de telle sorte, que l'élément de contact (13) soit placé de manière autoportante à l'intérieur d'une cellule (12) de la structure en treillis (10).

9. Dispositif selon l'une quelconque des revendications 1 à 7, la structure en treillis (10) comportant des connecteurs (20) dont chacun connecte l'une à l'autre quatre barrettes (11), l'élément de contact (13) étant placé directement sur un connecteur (20).

10. Dispositif selon l'une quelconque des revendications précédentes, l'élément de contact (13) comportant au moins deux électrodes (14) qui forment une paire d'électrodes bipolaire.

11. Dispositif selon la revendication 9, le capteur de température (15) étant placé entre deux électrodes (14) .

12. Dispositif selon l'une quelconque des revendications précédentes, la structure en treillis (10) comportant une matière à mémoire de forme, notamment un alliage nickel et titane, de préférence du nitinol.

13. Dispositif selon l'une quelconque des revendications précédentes, l'électrode (14) et / ou le capteur de température (15) et / ou la piste conductrice (18) comportant une matière biocompatible, conductrice d'électricité, notamment de l'or.

14. Système, doté d'un dispositif selon l'une quelconque des revendications précédentes et d'un système d'alimentation, notamment d'un cathéter (21) et d'un fil de transport (23), le fil de transport (23) étant guidé en étant déplaçable en longueur à l'intérieur du cathéter (21) et comportant un canal de passage destiné à recevoir un câble électrique et / ou un câble de données (27), qui connecte électriquement l'électrode (14) et / ou le capteur de température (15) avec un système de commande (26).
